# EUROPÄISCHE PATENTANMELDUNG

(11) **EP 0 965 325 A2**
(43) Veröffentlichungstag der Anmeldung: **22.12.1999**
(21) Anmeldenummer: 99110078.5
(22) Anmeldetag: 22.05.1999
(51) Int. Cl.: A61K 7/42

(54) **Dispersion anorganischer UV-Filter**

(30) Priorität: 16.06.1998 DE 19826840
(71) Anmelder: MERCK PATENT GmbH, 64293 Darmstadt (DE)
(72) Erfinder: Kurz, Thekla Dr., 64285 Darmstadt (DE); Hitzel, Sabine, 64409 Messel (DE); Wille, Dorothee, 64291 Messel (DE)

(57) **Zusammenfassung**

Die vorliegende Erfindung betrifft eine Dispersion von anorganischen UV-Filtern in flüssigen UV-Filtern und deren Verwendung in Sonnenschutzmitteln.

## Beschreibung

Die vorliegende Erfindung betrifft neue Dispersionen anorganischer UV-Filter und die Verwendung dieser Dispersionen in Sonnenschutzmitteln.

Während vor etwa 30 Jahren Sonnenlicht aufgrund der Vitamin D-Synthese als heilend und unbedenklich angesehen wurde, hat sich in den letzten Jahren die Einstellung in dieser Beziehung nicht nur aus medizinischer Sicht erheblich geändert. Das Gefahrenpotential, welches sowohl natürliche als auch künstliche Bestrahlung mit Sonnenlicht in sich birgt, ist im Bewußtsein in den Vordergrund gerückt. Insbesondere ist auch eine Verhaltensänderung hervorgerufen worden durch das Wissen über den Einfluß von Sonnenlicht auf die Hautalterung und die Entstehung von Hautkrebs.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten, und im extremen Fall kommt es zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Daher liegt das Hauptziel im Bereich Sonnenschutz eigentlich darin, einen guten Schutz gegen UVB- und UVA-Strahlung zu gewährleisten.

Innerhalb weniger Jahre haben anorganische Lichtschutzfilter einen festen Platz in der Sonnenkosmetik errungen.

Als geeignete anorganische Lichtschutzfilter seien hier Titandioxid, Zinkoxid, Eisenoxide oder auch Ceroxid genannt.

Mikropigmente, vor allem mikronisiertes Titandioxid, zeichnen sich durch ihre hohe Verträglichkeit und ihre besondere Stabilität aus. Über einen breiten UV-Bereich von 250 bis 380 nm kann mit Titandioxiden ein außerordentlich wirksamer Schutz erzielt werden.

Die anorganischen Filter werden im Sonnenschutz entweder als Pulver oder als Dispersionen in Öl oder Wasser eingesetzt. Mit Dispersionen werden dabei meist höhere Sonnenschutzfaktor-(SPF)-Werte erzielt, da durch eine Naßmahlung (z.B. in einer Perlmühle) ein höherer Dispersitätsgrad der Mikropigmente erreicht werden kann. Nachteilig ist jedoch hierbei, daß ein Hilfsstoff (das Dispersionsmedium) für den Endformulierer bereits vorgegeben ist. Das heißt, daß man bei der Herstellung von entsprechenden Sonnenschutzmitteln bei der Bestimmung des zu verwendenden Öles keine Wahl hat und meistens nicht ölfrei formulieren kann.

Ferner gibt es mit den bisher verwendeten anorganischen UV-Filtern stets Dispersionsprobleme, da sich die Partikel in den kosmetischen Formulierungen oftmals absetzen und daher eine optimale Anwendung auf der Haut nicht gewährleistet ist.

Aufgabe der Erfindung war es daher, eine Dispersion anorganischer UV-Filter zu finden, die bei der Herstellung von Formulierungen eine freie Wahl des zu verwendenden Öles gewährleistet und die eben beschriebenen Dispersionsprobleme löst.

Überraschernderweise wurde nun gefunden, daß die Dispersion von Titandioxid, Zinkoxid oder anderen anorganischen UV-Filtern in einem flüssigen UV-Filter die Lösung für die genannte Aufgabe darstellt.

Gegenstand der Erfindung ist daher eine Dispersion eines anorganischen UV-Filters, die dadurch gekennzeichnet ist, daß der anorganische UV-Filter in einem flüssigen UV-Filter dispergiert wird.

Als anorganische UV-Filter kommen dabei alle sonnenschutzwirksamen Pigmente in Frage. Vorzugsweise werden mikronisiertes Titandioxid, Zinkoxid, Ceroxid oder auch Eisenoxide verwendet.

Herkömmliche Titandioxide führten bisher zu einem starken Weißeln der Formulierungen auf der Haut.

Daher wird in einer besonders bevorzugten Ausführungsform dieser Erfindung als anorganischer UV-Filter mikronisiertes TiO₂ eingesetzt. Dieses ist als Eusolex® T-2000 (Merck KGaA, Darmstadt) im Handel zu beziehen.

Eusolex® T-2000 ist ein Titandioxid der zweiten Generation. Dieser UV-Filter ist ein Titandioxid auf Basis der Rutil-Modifikation und liegt in extrem kleinen Primärpartikeln vor. Durch diese Eigenschaften wird nicht nur ein hohes Absorptionsvermögen im UVB-Bereich, sondern auch im UVA-Bereich erzielt. Trotzdem ist die Transparenz im visuellen Spektrum sehr hoch. Der unerwünschte "Weißeffekt" tritt daher nicht auf.

Schon mit geringen Konzentrationen werden hohe Sonnenschutzfaktoren erzielt. Das Verhältnis von UVA zu UVB-Faktor liegt ohne weitere Zusätze etwa bei 0,5.

Eusolex® T-2000 kann problemlos sowohl in Wasser als auch in Öl oder Silikonöl eingearbeitet werden. Das ist zurückzuführen auf eine aufwendige Modifikation der Oberfläche, die dem Produkt amphiphile Eigenschaften verleiht.

Bei der erfindungsgemäßen Dispersion dient also ein flüssiger UV-Filter als Dispersionsmedium. Dies ist eine äußerst praktische Lösung, da anorganische UV-Filter ohnehin häufig mit anderen UV-Filtern kombiniert eingesetzt werden.

Als flüssige UV-Filter werden vorzugsweise Substanzen aus der Gruppe der Octocrylene (Eusolex® OCR), Octylmethoxycinnamate (Methoxyzimtsäureester, z.B. Eusolex® 2292), Homosalicylate (HMS), Octylsalicylat (z.B. Eusolex® OS) oder Octyl-dimethyl-p-aminobenzoesäure verwendet.

Vorzugsweise wird zur Stabilisierung der Dispersion ein Gelbildner zugegeben. Dies kann notwendig sein, da aufgrund des Dichteunterschiedes zwischen Pigment und organischem UV-Filter die Pigmente ohne den Zusatz von Gelbildnern meistens sedimentieren. Der Gelbildner hat jedoch praktisch keinen Einfluß auf die Endformulierung. Als Gelbildner kommen allgemein öldispergierbare Gerüstbildner in Frage. Bevorzugt werden in den erfindungsgemäßen Dispersionen Bentone, Aerosil oder Ethylcellulose verwendet.

Die Herstellung der Dispersionen erfolgt vorzugsweise in der Weise, daß man zunächst eine Mischung aus Pigment und Gelbildner vorbereitet, in welche man den UV-Filter dann in kleinen Teilen einarbeitet. Weiterhin ist es möglich, zunächst eine Mischung aus UV-Filter und Gelbildner herzustellen und anschließend das Pigment in diese Mischung einzuarbeiten.

Der Anteil an anorganischem UV-Filter in dem flüssigen UV-Filter beträgt 5 bis 80 Gew.-%, vorzugsweise 25 bis 50 Gew.-%.

Der Anteil an anorganischem UV-Filter kann sich entweder aus einem einzigen oder aus einer Kombination mehrerer anorganischen UV-Filtern zusammensetzen. Dies gilt ebenso für den Anteil an flüssigem UV-Filter.

Der Anteil an Gelbildner kann eigentlich beliebig gewählt werden, jedoch ist es sinnvoll, diesen Anteil so gering wie möglich zu halten. Vorzugsweise beträgt der Anteil bis zu 10 Gew.-%, insbesondere bevorzugt liegt der Anteil an Gerüstbildner bei ca. 5 Gew.-%.

Die erfindungsgemäßen Dispersionen erlauben nun dem Formulierer, Öle seiner Wahl einzusetzen oder sogar ölfrei zu formulieren. Es können hohe Sonnenschutzfaktor-Werte (SPF-Werte) erzielt werden, ohne Dispersionprobleme bei den anorganischen Pigmenten befürchten zu müssen.

Erfindungsgemäß können diese UV-Filter enthaltenden Dispersionen für sich allein oder natürlich auch in Kombination mit weiteren organischen oder anorganischen UVA- und UVB-Filtern oder deren Gemische in Sonnenschutzmitteln verwendet werden.

Gegenstand der Erfindung ist daher auch die Verwendung einer erfindungsgemäßen Dispersion in kosmetischen Formulierungen wie Sonnenschutzmitteln, Hautcremes- oder gelen, Haargelen oder kosmetischen Stiften.

Gegenstand der Erfindung sind auch solche Sonnenschutzmittel, die eine erfindungsgemäße Dispersion anorganischer UV-Filter in einem flüssigen UV-Filter enthalten.

Die erfindungsgemäße Dispersion kann in einer Konzentration von 0,5 bis 30 Gew.-%, vorzugsweise 5 bis 20 Gew.-%, in kosmetische Formulierungen eingearbeitet werden. Es ist auf diese Weise möglich Formulierungen herzustellen, in denen bis zu 100 % der eingesetzten Lichtschutzfilter in der erfindungsgemäßen Dispersion enthalten sind. Die erfindungsgemäßen Dispersionen können mit Wasser und Ölen in einfacher Weise gelöst, dispergiert oder emulgiert werden.

Die erfindungsgemäßen Dispersionen lassen sich direkt ohne weitere vorbereitende Maßnahmen in kosmetischen Formulierungen einarbeiten.

Diese Dispersionen bieten ferner den großen Vorteil, keine toxischen oder allergischen Reaktionen gegenüber der Haut zu zeigen.

Die erfindungsgemäßen kosmetischen Formulierungen weisen einen deutlich verbesserten Schutz gegen den schädlichen Einfluß von Sonnenstrahlen auf. Zudem treten keine Dispersionsprobleme, die bisher in Formulierungen enthaltend anorganische UV-Filter eine große Rolle spielen, auf.

Ebenso ist ein Verfahren zum Schutz der Haut vor Sonnenstrahlen, wobei auf die Haut eine erfindungsgemäße kosmetische Zubereitung aufgetragen wird, Gegenstand der Erfindung.

Eine Kombination der erfindungsgemäßen Dispersion mit weiteren organischen oder anorganischen UV-Filtern ist möglich und auch bevorzugt.

Als geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, beispielsweise seien hier nur Substanzen wie Benzylidencampherderivate (z.B. Eusolex ® 6300) oder Phenylbenzimidazol-5-sulfonsäure (Eusolex® 232), Benzoyl- oder Dibenzoylmethane wie Eusolex® 9020 oder Eusolex® 8020, Benzophenone (Eusolex® 4360), Methoxyzimtsäureester (z.B. Eusolex® 2292), Salicylatderivate (z.B. Eusolex® OS), Octocrylen (Eusolex® OCR), 4-Amino-benzoesäure (PABA), Homosalicylate (HMS) oder auch Octyl Triazone (Uvinul® T 150) aufgeführt.

Diese organischen UV-Filter werden in der Regel in einer Menge von 0,5 bis 10 Gewichtsprozent, vorzugsweise 1 - 8 %, in kosmetische Formulierungen eingearbeitet.

Als anorganische UV-Filter können dem Fachmann allgemein bekannte UV-Filter wie zum Beispiel solche aus der Gruppe Titandioxid und Zinkoxid eingesetzt werden. Diese anorganischen UV-Filter werden in der Regel in einer Menge von 0,5 bis 20 Gewichtsprozent, vorzugsweise 2 - 10 %, in kosmetische Formulierungen eingearbeitet.

Die erfindungsgemäßen Dispersionen weisen eine hohe chemische Stabilität, d.h. keine Hydrolysierbarkeit, keine Photooxidierbarkeit, keine Oxidierbarkeit, hohe Thermostabilität und hohe Schweißfestigkeit auf.

Gegebenenfalls können die erfindungsgemäßen Sonnenschutzmittel auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden. Solche können sowohl pflanzliche Extrakte als auch synthetische Selbstbräuner, wie z.B. Dihydroxyaceton oder α-Ketole, sein.

Weiterhin können die erfindungsgemäßen Formulierungen auch zur vorbeugenden Behandlung von Entzündungen und Allergien der Haut sowie auch in bestimmten Fällen zur Verhütung bestimmter Krebsarten verwendet werden.

Die erfindungsgemäße Zubereitung wird als Mittel zum Schutz der menschlichen Epidermis oder der Haare oder auch der sensibilisierten Haare oder als Sonnenschutzmittel verwendet.

Mit "sensibilisierten Haaren" sind Haare gemeint, welche einer Dauerwellenbehandlung, einem Färbe- oder Entfärbeprozeß unterzogen worden sein.

Die erfindungsgemäße kosmetische Zubereitung wird zum Schutz menschlicher Epidermis gegen Sonneneinstrahlung verwendet. Dabei liegt sie in verschiedenen, für diesen Typ üblicherweise verwendeten Formen vor. So kann sie insbesondere als Lotion oder Emulsion, wie als Creme oder Milch (O/W, W/O), oder in Form öliger oder ölig-alkoholischer Lotionen, Emulsionen, wie als Creme oder als Milch, in Form ölig-alkoholischer, ölig-wäßriger oder wäßrig-alkoholischer Gele oder als feste Stifte vorliegen oder als Aerosol konfektioniert sein.

Die Formulierung kann kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersionshilfs- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugt Ausführungsform der Erfindung ist eine Emulsion, welche als Schutzcreme oder -milch vorliegt und außer der erfindungsgemäßen Dispersion (enthaltend einen anorganischen UV-Filter in einem flüssigen UV-Filter) und gegebenenfalls noch weiteren Lichtschutzfiltern Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glykols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor Sonneneinstrahlung schützen, so kann es als Shampoo, Lotion, Gel oder Emulsion zum Ausspülen vorliegen, wobei die jeweilige Formulierung vor oder nach dem Schampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer den erfindungsgemäßen Dispersionen (enthaltend einen anorganischen UV-Filter in einem flüssigen UV-Filter) und gegebenenfalls weiteren Lichtschutzfiltern verschiedene, in diesem Mitteltyp verwendete Adjuvanzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele sollen die Erfindung näher erläutern.

### Beispiel 1

Aus folgenden Komponenten stellt man eine erfindungsgemäße Sonnenschutzcreme (W/O) mit einer UV-Filter-Suspension her.

| | | | Gew.-% |
|---|---|---|---|
| A | UV-Filter-Suspension (siehe C) | | 15,00 |
| | Eusolex® 9020 (Art.-Nr. 105844) | (1) | 2,00 |
| | Abil EM 90 | (2) | 3,00 |
| | Jojoba Öl | (3) | 5,00 |
| | Cetiol V | (4) | 1,00 |
| | Prisorine 2021 | (5) | 4,00 |
| | Castor Öl | (6) | 1,00 |
| | Lunacera W 80 | (7) | 1,50 |
| | Oxynex K flüssig (Art.-Nr. 108324) | (1) | 0,05 |
| B | Glycerin (Art-Nr. 104093) | (1) | 2,00 |
| | Natriumchlorid (Art.-Nr. 106400) | (1) | 0,40 |
| | Konservierungsmittel | | q.s. |
| | Wasser, demin. | | ad 100,00 |

Als Konservierungsmittel können
0.05 % Propyl-4-hydroxybenzoat (Art-Nr. 107427) oder
0.15 % Methyl-4-hydroxybenzoat (Art-Nr. 106757)
verwendet werden.

| **C UV-Filter-Suspension** | | |
|---|---|---|
| Eusolex® T-2000 (Art-Nr. 105373) | (1) | 30,00 % |
| Eusolex® OCR (Art-Nr. 105377) | (1) | 65,00 % |
| Aerosil R 812 | (8) | 5,00 % |

Man mischt Eusolex® T-2000 zunächst mit dem Aerosil und gibt anschließend das Eusolex® OCR zu. Man rührt bis man eine homogene Mischung erhält.

### Herstellung:

Die Komponenten der Phase A werden gemischt und auf 75 °C erwärmt. In diese Phase wird die vorgemischte Phase B unter Rühren eingebracht. Man rührt bis man eine homogene Mischung hat und läßt unter Rühren abkühlen.

### Bezugsquellen:

(1) Merck KGaA, Darmstadt
(2) Th. Goldschmidt, Essen
(3) Lamotte, Bremen
(4) Henkel KGaA, Düsseldorf
(5) Unichema, Emmerich
(6) Heess, Stuttgart
(7) Fuller, Lüneburg
(8) Degussa, Frankfurt

### Beispiel 2

Aus folgenden Komponenten stellt man eine erfindungsgemäße Sonnenschutzcreme (W/O) mit einer UV-Filter-Suspension her.

| | | | Gew.-% |
|---|---|---|---|
| A | UV-Filter-Suspension (siehe C) | | 15,00 |
| | Eusolex® 9020 (Art-Nr. 105844) | (1) | 2,00 |
| | Abil EM 90 | (2) | 3,00 |
| | Jojoba Öl | (3) | 5,00 |
| | Cetiol V | (4) | 1,00 |
| | Prisorine 2021 | (5) | 4,00 |
| | Castor Öl | (6) | 1,00 |
| | Lunacera W 80 | (7) | 1,50 |
| | Oxynex K flüssig (Art-Nr. 108324) | (1) | 0,05 |
| B | Glycerin (Art-Nr. 104093) | (1) | 2,00 |
| | Natriumchlorid (Art-Nr. 106400) | (1) | 0,40 |
| | Konservierungsmittel | | q.s. |
| | Wasser, demin. | | ad 100,00 |

Als Konservierungsmittel können
0.05 % Propyl-4-hydroxybenzoat (Art-Nr. 107427) oder
0.15 % Methyl-4-hydroxybenzoat (Art-Nr. 106757)
verwendet werden.

| **C UV-Filter-Suspension** | | |
|---|---|---|
| Eusolex® T-2000 (Art-Nr. 105373) | (1) | 30,00 % |
| Eusolex® OCR (Art.-Nr. 105377) | (1) | 65,00 % |
| Bentone Paste SIL | (8) | 5,00 % |

Man mischt die Bentone Paste und das Eusolex OCR, um die Paste zu dispergieren. Dann gibt man Eusolex T-2000 sukzessive hinzu und rührt bis die Suspension homogen ist.

### Herstellung:

Die Komponenten der Phase A werden gemischt und auf 75 °C erwärmt. In diese Phase wird die vorgemischte und auf 80 °C erwärmte Phase B unter Rühren eingebracht. Man rührt bis man eine homogene Mischung hat und läßt unter Rühren abkühlen.

### Bezugsquellen:

(1) Merck KGaA, Darmstadt
(2) Th. Goldschmidt, Essen
(3) Lamotte, Bremen
(4) Henkel KGaA, Düsseldorf
(5) Unichema, Emmerich
(6) Heess, Stuttgart
(7) Fuller, Lüneburg
(8) Kronos Titan, Leverkusen

### Beispiel 3

Aus folgenden Komponenten stellt man eine erfindungsgemäße Sonnenschutzcreme (W/O) mit einer UV-Filter-Suspension her.

| | | | Gew.-% |
|---|---|---|---|
| A | UV-Filter-Suspension (siehe C) | | 10,00 |
| | Eusolex® 6300 (Art-Nr. 105385) | (1) | 3,00 |
| | Dow Corning 3225 C | (2) | 12,00 |
| | Dow Corning 344 | (2) | 2,50 |
| | Solvent ID | (3) | 7,30 |
| | Bentone Paste SIL | (6) | 14,50 |
| | Witconol 14 | (4) | 2,50 |
| | Bienenwachs, weiß (Art-Nr. 111544) | (1) | 2,00 |
| | Carnauba Wachs | (5) | 0,50 |
| B | Propandiol-1,2 (Art.-Nr. 107478) | (1) | 2,00 |
| | Natriumchlorid (Art-Nr. 106400) | (1) | 2,00 |
| | Konservierungsmittel | | q.s. |
| | Wasser, demin. | | ad 100,00 |

Als Konservierungsmittel kann
0,20 % Euxyl K 400 (Schülke & Mayr, Norderstedt)
verwendet werden.

| **C UV-Filter-Suspension** | | |
|---|---|---|
| Eusolex® T-2000 (Art-Nr. 105373) | (1) | 25,00 % |
| Eusolex® 2292 (Art-Nr. 105382) | (1) | 70,00 % |
| Bentone Paste SIL | (6) | 5,00 % |

Man mischt die Bentone Paste und das Eusolex 2292, um die Paste zu dispergieren. Dann gibt man Eusolex T-2000 sukzessive hinzu und rührt bis die Suspension homogen ist.

### Herstellung:

Die Phasen A und B werden jeweils gemischt und auf 80 °C erwärmt. Phase B wird dann in die Phase A eingetragen. Man rührt bis man eine homogene Mischung hat und läßt unter Rühren abkühlen.

### Bezugsquellen:

(1) Merck KGaA, Darmstadt
(2) Dow Corning, Düsseldorf
(3) BP, Düsseldorf
(4) Witco Chemical, Frankfurt
(5) Aug. Schmidt Nachfolger, Bremen
(6) Kronos Titan, Leverkusen

## Patentansprüche

1. Dispersion anorganischer UV-Filter, dadurch gekennzeichnet, daß die anorganischen UV-Filter in einem flüssigen UV-Filter dispergiert sind.

2. Dispersion nach Anspruch 1, dadurch gekennzeichnet, daß als anorganische UV-Filter mikronisiertes Titandioxid, Zinkoxid, Ceroxid oder Eisenoxide enthalten sind.

3. Dispersion nach Anspruch 1 oder 2, dadurch gekennzeichnet, daß als flüssige UV-Filter Substanzen aus der Gruppe der Octocrylene, Octylmethoxycinnamate, Homosalicylate, Octylsalicylat oder Octyldimethyl-p-aminobenzoesäure enthalten sind.

4. Dispersion nach einem der Ansprüche 1 bis 3, dadurch gekennzeichnet, daß zur Stabilisierung Gelbildner oder Dispersionshilfsmittel enthalten sind.

5. Dispersion nach einem der Ansprüche 1 bis 4, dadurch gekennzeichnet, daß der Anteil anorganischer UV-Filter in löslichem UV-Filter 5 bis 80 Gew.-% beträgt.

6. Verwendung einer Dispersion nach einem der Ansprüche 1 bis 5 in kosmetischen Formulierungen wie Sonnenschutzmitteln, Hautcremes oder -gelen, Haargelen oder kosmetischen Stiften.

7. Verwendung einer Dispersion nach einem der Ansprüche 1 bis 5 in Kombination mit weiteren organischen oder anorganischen UVA- und UVB-Filtern.

8. Sonnenschutzmittel, dadurch gekennzeichnet, daß es eine Dispersion nach einem der Ansprüche 1 bis 5 enthält.
